# EUROPEAN PATENT SPECIFICATION

(11) **EP 0 994 113 B1**
(45) Date of publication and mention of the grant of the patent: **18.08.2004**
(21) Application number: 99120013.0
(22) Date of filing: 15.10.1999
(51) Int. Cl.: C07D 471/04, A61K 31/505, A61P 11/08

(54) **7-Aminopyrido(2,3-d)-pyrimidine derivatives for treatment of bronchial asthma**
7-Aminopyrido(2,3-d)-pyrimidine Derivate zur Behandlung von Bronchialasthma
Dérivés de 7-aminopyrido(2,3-d)-pyrimidine pour traitement de l'asthme bronchique

(30) Priority: 15.10.1998 JP 29325098
(43) Date of publication of application: 19.04.2000
(73) Proprietor: NIPPON ZOKI PHARMACEUTICAL CO., LTD., Chuo-ku, Osaka (JP)
(72) Inventor: Ogino, Takashi, Inst. Bio-Active Science, Yashiro-cho, Katoh-gun, Hyogo (JP); Furukuwa, Kazuhito, Inst. Bio-Active Science, Yashiro-cho, Katoh-gun, Hyogo (JP)
(74) Representative: HOFFMANN - EITLE

(56) References cited:
- EP-A- 0 163 599
- EP-A- 0 260 817
- EP-A- 0 696 590
- WO-A-93/19068
- US-A- 3 272 816
- MULLER T ET AL: "Subtypes of the type 4 cAMP phosphodiesterases: structure, regulation and selective inhibition" TRENDS IN PHARMACOLOGICAL SCIENCES,GB,ELSEVIER TRENDS JOURNAL, CAMBRIDGE, vol. 17, no. 8, 1 August 1996 (1996-08-01), pages 294-298, XP004034578 ISSN: 0165-6147

## Description

### Technical Field of the Invention

The present invention relates to a novel 7-amino-1-phenylpyrido[2,3-d]pyrimidine-2,4-dione derivative and a medical use thereof.

### Prior Art

Allergy is a pathological condition where living body is damaged by an immune response though the response is originally a biological defense reflex. Allergic rhinitis is divided broadly into two groups. Namely, one is perennial rhinitis caused by house dust or by mite and another is pollinosis linked with a lot of pollen flying whereby many patients are generated as well. There is a difference in symptoms between those allergies and, therefore, therapeutic method is different. But, when once a person is suffered from the disease, natural healing at an early stage cannot be expected and no therapeutic method to cure completely has been established yet. Therefore, numbers of patients are cumulatively increasing.

Bronchial asthma is a disease which is characterized in a paroxysmal dyspnea accompanied by coughs and wheezes. Although its cause is ambiguous, a concept that it is a chronic inflammatory disease of airway has been established recently in addition to the already proposed concept of reversible obstructive impairment and hypersensitivity of airway. Accordingly, in the current therapy, steroidal preparations are used with an object of suppression of inflammation of airway and, since the diseases is also accompanied by an airway obstruction, anti-chemical mediators or bronchial dilators are used jointly.

Steroidal preparations used by means of inhalation, oral administration, intravenous injection, etc. are the pharmaceuticals which express various side effects together with a sharp clinical effect and, with regard to main side effects, induction of infectious diseases, osteoporosis, arteriosclerosis, diabetes mellitus, mental disorder and moon face are known. It is said that serious side effect occurs when administration of steroidal preparation extends over a long period and that frequency and degree of seriousness of adrenal insufficiency is dependent upon the dose and term of the administration. Especially, the withdrawal symptom occurred by a rapid reduction of the administering dose and the adrenal insufficiency by adrenal cortical shrinkage due to administration of high dose for a long period are said to be problems.

Anti-chemical mediators are the pharmaceuticals which inhibit the biosynthesis and liberation of chemical mediators participating in allergy such as histamine, thromboxane and leukotriene, or the pharmaceuticals which antagonize the binding of such chemical mediators to the receptors. Thus, such anti-chemical mediators are not the direct therapeutic agents for dilating the shrunk airway of asthma and for improving the dyspnea but are used as the pharmaceuticals for preventing the onset of asthma symptoms caused by chemical mediators.

Bronchialdilators are β₂ stimulants and theophylline preparations which are used as rapid-acting therapeutic agents for relieving the dyspnea symptom upon asthma and, in the case of onset of severe asthma, therapies such as a subcutaneous injection of β ₂ stimulant and a continuously intravenous drip of theophylline are carried out. However, in the treatment of β₂ stimulant, there is a problem of death by suffocation from a negative feedback due to its abuse. The theophylline preparation also has a disadvantage that its safety region is narrow and that, at high concentrations, occurrence of toxic symptoms, headache, vomiting, pulsation and extrasystole takes place. Because of those reasons, at present, caution for abuse is required for β₂ stimulants, and a therapeutic drug monitoring (TDM) is carried out for theophylline preparations.

As mentioned above, the already-known therapeutic agents for bronchial asthma have both merits and demerits in terms of onset of the effect and generation of the side effect. And, therefore, in the practical clinical field, there has been a demand for the pharmaceuticals having higher safety and more rapidly acting property.

It has been reported already that the known compounds having a similar pyrido[2,3-d]pyrimidine structure to the compounds of the present invention have an anti-allergic action (Japanese Laid-Open Patent Publication Sho-63/45279). It has been also known that the compounds having a 7-aminopyrido[2,3-d]pyrimidine structure show a bronchial dilating action (Japanese Laid-Open Patent Publications Hei-8/3046, Hei-8/3164 and Hei-8/3165). However, in those known compounds, separation of pharmaceutical effect from side effect is not sufficient and, with regard to the bronchial dilating action, they are not satisfactory as well whereby they have not been allowed as the pharmaceuticals for actual use. Besides the above-mentioned ones, various compounds having a pyrido[2,3-d]pyrimidine structure have been reported (refer, for example, to Japanese Laid-Open Patent Publication Hei-7/504676; *Cell Signals*, **7**, 527 (1995); Mol. Pharmacol., **48,** 616 (1995); *J.Med*. *Chem*., **34**, 624 (1991); and *J. Pharmacol. Exp. Ther.*, **272,** 1313 (1995)). However, those compounds have a problem in terms of their behavior *in vivo* such as poor transfer into blood and none of them have been put in the market as pharmaceuticals. Incidentally, there has been no report at all for the 7-amino-1-phenylpyrido[2,3-d]-pyrimidine-2,4-dione derivatives of the present invention.

### Problem and Solution

The problem underlying the invention of the present application is to solve the above-mentioned problems in prior art and is to offer a therapeutic agent for bronchial asthma which has been briskly demanded by patients and by medical fields, i.e. the agent having a high safety, a rapidly acting property and a good behavior *in vivo.*

The present inventors have carried out an intensive investigation for 7-aminopyrido[2,3-d]pyrimidine derivatives and have found that 7-amino-1-phenylpyrido[2,3-d]-pyrimidine-2,4-dione derivatives have an excellent bronchial dilating action, have high safety and little side effect and exhibit good behavior *in vivo* whereupon the present invention has been achieved. Consequently, the compounds of the present invention are very useful as therapeutic agents for bronchial asthma.

### Best Mode for Carrying Out the Invention

The present invention relates to 7-amino-1-phenylpyrido[2,3-d]pyrimidine-2,4-dione derivatives represented by the following formula (I) and pharmaceutically acceptable salts and hydrates thereof and it further relates to a therapeutic agent for bronchial asthma containing the said compound as an effective component. 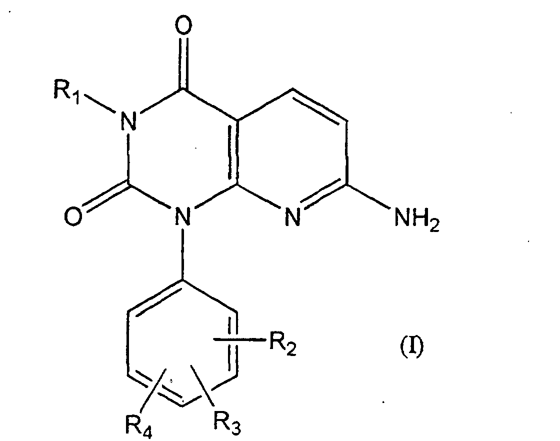 wherein
R₁ is C₂₋₆-alkenyl, phenyl, C₃₋₆-alkyl or C₁₋₆-alkyl which is substituted with a substituent selected from
(a) oxo,
(b) C₁₋₆-alkoxy,
(c) phenyl which is optionally substituted with one or more C₁₋₆-alkyl, C₁₋₆-alkoxy, carboxyl, (C₁₋₆-alkoxy)carbonyl, mercapto, halogen, trifluoromethyl and/or nitro;
(d) naphthyl,
(e) furyl,
(f) isoxazolyl which is optionally substituted with one or more C₁₋₆-alkyl,
(g) pyridyl which is optionally substituted with one or more C₁₋₆-alkyl and/or halogen.
(h) thienyl which is optionally substituted with halogen, and
(i) 1,3-dioxolanyl;
and R₂, R₃ and R₄ each independently is hydrogen, halogen, C₁₋₆alkoxy, benzyloxy, carboxyl or (C₁₋₆-alkoxy)carbonyl.

In the formula, C₁₋₆-alkyl means a linear or branched C₁₋₆-alkyl such as methyl, ethyl, propyl, isopropyl, butyl, isobutyl, sec-butyl or t-butyl, pentyl, isopentyl, neopentyl, t-pentyl, hexyl, isohexyl or dimethylbutyl.

Also, C₁₋₆-alkoxy represents a linear or branched C₁₋₆-alkoxy such as methoxy, ethoxy, propoxy, isopropoxy, butoxy, isobutoxy, sec-butoxy or t-butoxy, pentyloxy, isopentyloxy, neopentyloxy, t-pentyloxy, hexyloxy, isohexyloxy or dimethylbutoxy.

"Halogen" preferably is a fluorine, chlorine, bromine or iodine atom.

Finally, C₂₋₆-alkenyl means a linear or branched C₂₋₆-alkenyl such as ethenyl, propenyl, butenyl, pentenyl or hexenyl.

Preferred embodiments of the present invention are given as follows.
- (1): A 7-amino-1-phenylpyrido[2,3-d]pyrimidine-2,4-dione derivative of the above formula (I) and pharmaceutically acceptable salts and hydrates thereof.
- (2): A compound according to (1) wherein R₂ is hydrogen.
- (3): A compound according to (2) wherein R₃ is C₁₋₆-alkoxy
- (4): A compound according to (3) wherein R₃ is present in the meta-position of the aromatic ring.
- (5): A compound according to any of (3) to (4) wherein alkoxy is methoxy
- (6): A compound according to any of (3) to (5) wherein R₄ is C₁₋₆-alkoxy.
- (7): A compound according to (6) wherein R₄ is present in the meta-position of the aromatic ring.
- (8): A compound according to any of (6) to (7) wherein alkoxy is methoxy.
- (9): A compound according to any of (1) to (8) wherein R₁ is C₃₋₆-alkyl.
- (10): A compound according to (9) wherein alkyl is isobutyl.
- (11): A therapeutic agent for bronchial asthma comprising a compound according to any of (1) to (10) as an effective component.
- (12): A bronchial dilator comprising a compound according to any of (1) to (10) as an effective component.

Especially, preferred compounds of the present invention are given as follows (the numbering of the compounds will be adhered to later on where applicable).
- (1): 7-amino-1,2,3,4-tetrahydro-1,3-diphenylpyrido[2,3-d]pyrimidine-2,4-dione
- (3): 7-amino-1,2,3,4-tetrahydro-1-phenyl-3-propylpyrido[2,3-d]pyrimidine-2,4-dione
- (4): 7-amino-3-butyl-1,2,3,4-tetrahydro-1-phenylpyrido[2,3-d]pyrimidine-2,4-dione
- (6): 7-amino-1-(3,5-dimethoxyphenyl)-1,2,3,4-tetrahydro-3-propylpyrido[2,3-d] pyrimidine-2,4-dione
- (7): 7-amino-3-butyl-1-(3,5-dimethoxyphenyl)-1,2,3,4-tetrahydropyrido[2,3-d] pyrimidine-2,4-dione
- (8): 7-amino-3-benzyl-1-(3,5-dimethoxyphenyl)-1,2,3,4-tetrahydropyrido[2,3-d] pyrimidine-2,4-dione
- (9): 7-amino-1,2,3,4-tetrahydro-1-(4-methoxyphenyl)-3-propylpyrido[2,3-d] pyrimidine-2,4-dione
- (10): 7-amino-3-butyl-1,2,3,4-tetrahydro-1-(4-methoxyphenyl)pyrido[2,3-d] pyrimidine-2,4-dione
- (11): 7-amino-3-benzyl-1,2,3,4-tetrahydro-1-(4-methoxyphenyl)pyrido[2,3-d] pyrimidine-2,4-dione
- (12): 7-amino-1,2,3,4-tetrahydro-1-(4-methoxyphenyl)-3-(4-picolyl)pyrido[2,3-d] pyrimidine-2,4-dione
- (13): 7-amino-1-(3,4-dimethoxyphenyl)-1,2,3,4-tetrahydro-3-propylpyrido[2,3-d] pyrimidine-2,4-dione
- (14): 7-amino-1-(2,5-dimethoxyphenyl)-1,2,3,4-tetrahydro-3-propylpyrido[2,3-d] pyrimidine-2,4-dione
- (15): 7-amino-1-(3,5-dimethoxlphenyl)-1,2,3,4-tetrahydro-3-propylpyrido[2,3-d] pyrimidine-2,4-dione
- (16): 7-amino-3-benyzyl-1,2,3,4-tetrahydro-1-phenylpyrido[2,3-d]pyrimidine-2,4-dione
- (17): 7-amino-1,2,3,4-tetrahydro-1-phenyl-3-(4-picolyl)pyrido[2,3-d]pyrimidine-2,4-dione
- (18): 7-amino-1-(3,5-dimethoxyphenyl)-1,2,3,4-tetrahydro-3-(4-picolyl)pyrido[2,3-d] pyrimidine-2,4-dione
- (19): 7-amino-3-benzyl-]-(2,4-dimethoxyphenyl)-1,2,3,4-tetrahydropyrido[2,3-d] pyrimidine-2,4-dione
- (20): 7-amino-1-(3,5-dimethoxyphenyl)-3-(2-ethoxyethyl)-1,2,3,4-tetrahydropyrido[2,3-d]pyrimidine-2,4-dione
- (21): 7-amino-3-(3-butenyl)-1-(3,5-dimethoxyphenyl)-1,2,3,4-tetrahydropyrido[2,3-d] pyrimidine-2,4-dione
- (22): Methyl [7-amino-1,2,3,4-tetrahydro-3-(4-picolyl)-2,4-dioxopyrido[2,3-d] pyrimidine-1-yl]-3-benzoate
- (23): 7-amino-3-(4-chlolobenzyl)-1,2,3,4-tetrahydro-1-phenylpyrido[2,3-d] pyrimidine-2,4-dione
- (24): 7-amino-1,2,3,4-tetrahydro-3-(2-methylpicolyl)-1-phenylpyrido[2,3-d] pyrimidine-2,4-dione
- (25): 7-amino-1,2,3,4-tetrahydro-3-(2-picolyl)-1-phenylpyrido[2,3-d]pyrimidine-2,4-dione
- (26): 7-amino-1,2,3,4-tetrahydro-3-(3-picolyl)-1-phenylpyrido[2,3-d]pyrimidine-2,4-dione
- (27): 7-amino-3-(3-chlolobenzyl)-1,2,3,4-tetrahydro-1-phenylpyrido[2,3-d] pyrimidine-2,4-dione
- (28): 7-amino-1,2,3,4-tetrahydro-3-(4-methoxybenzyl)-1-phenylpyrido[2,3-d] pyrimidine-2,4-dione
- (29): 7-amino-3-(4-fluorobenzyl)-1,2,3,4-tetrahydro-1-phenylpyrido[2,3-d] pyrimidine-2,4-dione
- (30): 7-amino-1,2,3,4-tetrahydro-3-(4-methybenzyl)-1-phenylpyrido[2,3-d] pyrimidine-2,4-dione
- (31): 7-amino-1,2,3,4-tetrahydro-3-(3-nitrobenzyl)-1-phenylpyrido[2,3-d]pyrimidine-2,4-dione
- (32): 7-amino-3-(2-chlorobenzyl)-1,2,3,4-tetrahydro-1-phenylpyrido[2,3-d] pyrimidine-2,4-dione
- (33): 7-amino-1,2,3,4-tetrahydro-3-(3-methybenzyl)-1-phenylpyrido[2,3,d] pyrimidine-2,4-dione
- (34): 7-amino-3-(3,4-dichlorobenzyl)-1,2,3,4-tetrahydro-1-phenylpyrido[2,3-d] pyrimidine-2,4-dione
- (35): 7-amino-1,2,3,4-tetrahydro-3-(3-methoxybenzyl)-1-phenylpyrido[2,3-d] pyrimidine-2,4-dione
- (36): 7-amino-1,2,3,4-tetrahydro-3-(4-trifluoromethylbenzyl)-1-phenylpyrido[2,3-d] pyrimidine-2,4-dione
- (37): 7-amino-1,2,3,4-tetrahydro-1-phenyl-3-(thienylmethyl)pyrido[2,3-d]pyrimidine-2,4-dione
- (38): 7-amino-3-(2-furfuryl)-1,2,3,4-tetrahydro-1-phenylpyrido[2,3-d]pyrimidine-2,4-dione
- (39): 7-amino-1,2,3,4-tetrahydro-1-phenyl-3-(3-thienylmethyl)pyrido[2,3-d] pyrimidine-2,4-dione
- (40): 7-amino-3-(2-chloro-6-methylpicolyl)-1,2,3,4-tetrahydro-1-phenylpyrido[2,3-d] pyrimidine-2,4-dione
- (41): Methyl 4-[7-amino-1,2,3,4-tetrahydro-1-phenyl-2,4-dioxopyrido[2,3-d] pyrimidine-3-yl-methyl]benzoate
- (42): 7-amino-3-(2-dioxolanylmethyl)-1,2,3,4-tetrahydro-1-phenylpyrido[2,3-d] pyrimidine-2,4-dione
- (43): 4-[7-amino-1,2,3,4-tetrahydro-1-phenyl-2,4-dioxopyrido[2,3-d]pyrimidine-3-yl-methyl]benzoic acid
- (44): 7-amino-3-benzyl-1-(3-chlorophenyl)-1,2,3,4-tetrahydropyrido[2,3-d] pyrimidine-2,4-dione
- (45): 7-amino-1,2,3,4-tetrahydro-3-(4-nitrobenzyl)-1-phenylpyrido[2,3-d]pyrimidine-2,4-dione
- (46): 7-amino-1,2,3,4-tetrahydro-3-(2-methoybenzyl)-1-phenylpyrido[2,3-d] pyrimidine-2,4-dione
- (47): 7-amino-3-(3,5-dimethoxybenzyl)-1,2,3,4-tetrahydro-1-phenylpyrido[2,3-d] pyrimidine-2,4-dione
- (48): 7-amino-3-(5-chlorothienylmethyl)-1,2,3,4-tetrahydro-1-phenylpyrido[2,3-d] pyrimidine-2,4-dione
- (49): 7-amino-3-benzyl-1-(3,5-difluorophenyl)-1,2,3,4-tetrahydropyrido[2,3-d] pyrimidine-2,4-dione
- (50): 7-amino-1,2,3,4-tetrahydro-3-(1-naphthylmethyl)-1-phenylpyrido[2,3-d] pyrimidine-2,4-dione
- (51): 7-amino-1,2,3,4-tetrahydro-3-(3,5-dimethylbenzyl)-1-phenylpyrido[2,3-d] pyrimidine-2,4-dione
- (52): 7-amino-3-benzyl-1,2,3,4-tetrahydro-1-(3-methoxypheny)lpyrido[2,3-d] pyrimidine-2,4-dione
- (53): 7-amino-3-(4-bromobenzyl)-1,2,3,4-tetrahydro-1-phenylpyrido[2,3-d] pyrimidine-2,4-dione
- (54): 7-amino-3-(2-chloropicolyl)-1,2,3,4-tetrahydro-1-phenylpyrido[2,3-d] pyrimidine-2,4-dione
- (55): 7-amino-3-benzyl-1-(3-benzylovyphenyl)-1,2,3,4-tetrahydropyrido[2,3-d] pyrimidine-2,4-dione
- (56): 7-amino-1,2,3,4-tetrahydro-3-(3-methylisoxazol-5-yl-methyl)-1-phenylpyrido[2,3-d]pyrimidine-2,4-dione
- (57): 7-amino-3-(3,5-dimethylisoxazol-4-yl-methyl)-1,2,3,4-tetrahydro-1-phenylpyrido[2,3-d]pyrimidine-2,4-dione
- (58): 7-amino-1,2,3,4-tetrahydro-1-phenyl-3-(5-phenylpentyl)pyrido[2,3-d] pyrimidine-2,4-dione

In the above compounds of the present invention, the most preferred compound is 7-amino-1-(3,5-dimethoxyphenyl)-1,2,3,4-tetrahydro-3-propylpyrido[2,3-d]pyrimidine-2,4-dione (Compound 15).

The compounds of the present invention may be manufactured according to a method described in Japanese Laid-Open Patent Publication Sho-63/45279, Hei-8/3046, Hei-8/3164 or Hei-8/3165, and it will be further illustrated in detail by way of the following examples.

The compounds represented by the above-given formula (I) include the pharmaceutically acceptable salts of thereof such as acid addition salts with hydrochloric acid, sulfuric acid, nitric acid, hydrobromic acid, phosphoric acid, perchloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid or sulfanilic acid; salts with alkali metal such as sodium or potassium, salts with alkaline-earth metal such as calcium or magnesium, or salts with other metals such as aluminum; or salts with bases such as ammonia or organic amines. Those salts may be manufactured by known methods from the compounds of the present invention in a free state or may be mutually converted among the salts. When there are steric isomers such as cis-trans isomer, optical isomer, conformational isomer and hydrate for the substances of the present invention, the present invention includes any and all of them.

The substance of the present invention can be made into pharmaceutical preparations by a combination with a suitable pharmaceutical carriers or diluents. Any of the known methods for providing preparations, such as for oral or parenteral administrations (e.g. solids, half-solids, liquids or gases) may be used to produce the pharmaceutical compositions of the present invention. In preparing the preparations, the substance of the present invention may be used in the form of their pharmaceutically acceptable salts, and also can be used either solely or jointly together with other pharmaceutically active components.

In the case of preparations for oral administration, the substance of the present invention as it is or together with commonly-used excipients such as a suitable additive (e.g. lactose, mannitol, corn starch, potato starch, etc.) is mixed with binders such as crystalline cellulose, cellulose, gum arabicum, corn starch, gelatin, etc., disintegrating agents such as corn starch, potato starch, potassium carboxymethylcellulose, etc., lubricating agents such as talc, magnesium stearate, etc. and others including bulking agents, moisturizing agents, buffers, preservatives, perfumes and the like to give tablets, diluted powders, granules or capsules.

Alternatively, suppositories may be prepared by mixing with fatty/oily bases (e.g. cacao butter), emulsified bases, water-soluble bases (e.g. macrogol), hydrophilic bases, etc.

In the case of injections, it is possible to prepare the solutions or the suspensions in an aqueous and nonaqueous solvents such as distilled water for injection, physiological saline solution, Ringer's solution, plant oil, synthetic fatty acid glycerides, higher fatty acid esters, propylene glycol, etc.

In case of inhalations or aerosol preparations, the compound of the present invention in the form of a liquid or minute powder can be filled up in an aerosol container with gas or liquid spraying agent, and if desired, with conventional adjuvants such as humidifying agents or dispersing agent. They can also be used as pharmaceuticals for a non-pressurized preparation such as in a nebulizer or an atomizer. It is also possible, depending upon the type of the disease, to prepare the pharmaceutical preparations which are other than those which were mentioned already and are suitable for the therapy such as, for example, collyriums, ointments, poultices, etc.

The preferred dose of the compound of the present invention may vary depending upon the object to be administered the patient, form of the preparation, method for the administration, term for the administration, etc. and, in order to achieve a desired effect, 0.001-50 mg per day, preferably 0.05-25 mg per day may be usually given to common adults by oral route. In the case of a parenteral administration such as by injection, it is preferred that, due to the influence of the absorption, etc., a level of from 1/3 to 1/10 of the above-given dose by oral route is administered.

The present invention will be further illustrated by way of the following examples although the present invention is not limited by them at all.

### Examples

The starting materials may be purchased from Aldrich Chemical Co., Inc.; Furuka Chemical Inc.; Lancaster Synthesis Inc.; Maybridge Chemical Co., Ltd.; or Tokyo Kasei K.K. or may be synthesized by known methods mentioned in literatures such as *J. Org. Chem*., **16**, 1879 (1951);*J. Am. Chem. Soc.*, **75**, 114 (1953); etc.

### Example 1

### (1) Manufacture of 6-amino-5-formyl-1,3-diphenyluracil.

A solution of 6-amino-1,3-diphenyluracil (10.0 g, 35.8 mmol) in dimethylformamide (100 mL) was cooled in an ice bath and phosphorus oxychloride (3.7 mL, 39.4 mmol) was dropped thereinto using a dropping funnel. The reaction mixture was stirred at room temperature for two hours and the reaction was stopped by adding 50 mL of water thereto. The pH was adjusted to 10 by a 1N solution of potassium hydroxide and stirring was carried out at room temperature for additional one hour. Crude crystals separated out therefrom were filtered and, after washing with 100 mL of water, the crude crystals were filtered. The resulting crude crystals were further recrystallized from hexane and ethyl acetate to give 6-amino-5-formyl-1,3-diphenyluracil (2.2 g) in a 40% yield (Mp: 141-142°C).
¹H-NMR (DMSO-d₆) d: 7.29-7.61 (m, 10H), 9.80 (s, 1H), 9.98 (s, 1H)
IR (KBr): 3309, 1730, 1662, 1647, 1616, 1516, 1491, 1365, 770, 692 cm⁻¹

| | | | | |
|---|---|---|---|---|
| Analysis | Calculated for C₁₇H₁₃N₃O₃ | C, 66.44; | H, 4.26; | N, 13.67 |
| | Found | C, 66.59; | H, 4.24; | N, 13.77 |

MS (EI) m/z: 307 [M⁺], 279, 160, 132, 77

### (2) Manufacture of 7-amino-1,2,3,4-tetrahydro-1,3-diphenylpyrido[2,3-d]pyrimidine-2,4-dione (Compound 1).

A solution of 6-amino-5-formyl-1,3-diphenyluracil (5.0 g, 16.3 mmol) and 2-(triphenylphosphoranylidene)acetonitrile (5.9 g, 19.6 mmol) in anhydrous acetonitrile (100 mL) was heated to reflux for 24 hours in an argon stream. The reaction mixture was allowed to cool and the solvent was evaporated therefrom *in vacuo*. The crude crystals separated out therefrom were recrystallized from benzene to give 7-amino-1,2,3,4-tetrahydro-1,3-diphenylpyrido[2,3-d]pyrimidine-2,4-dione (2.4 g) in a 45% yield (Mp: 162-163°C).
¹H-NMR (DMSO-d₆) d: 6.33 (d, 1H, J=9Hz), 6.89 (br, 2H), 7.31-7.93 (m, 10H), 7.93 (d, 1H, J=9Hz)
IR (KBr): 3358, 1709, 1660, 1624, 1427, 1398, 694 cm⁻¹

| | | | | |
|---|---|---|---|---|
| Analysis | Calculated for C₁₉H₁₄N₄O₂ | C, 69.08; | H, 4.27; | N, 16.96 |
| | Found | C, 68.99; | H, 4.37; | N, 16.97 |

MS (EI) m/z: 330 [M⁻], 211

Appropriate starting materials were used in place of 6-amino-1,3-diphenyluracil which was the starting material in the above Example 1 and subjected to a method mentioned in Example 1 in the same manner whereupon the Compounds 2 to 58 represented by the following formula (II) were manufactured. Details of the compounds are mentioned in Table 1.

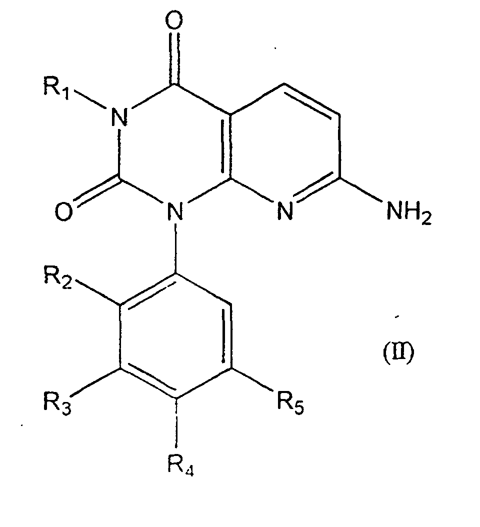

**Table 1**

| Comp. No. | R₁ | R₂ | R₃ | R₄ | R₅ | Mp (°C) |
|---|---|---|---|---|---|---|
| 3 | Pr | H | H | H | H | 198 - 199 |
| 4 | Bu | H | H | H | H | 209 - 211 |
| 5* | Et | H | OMe | H | OMe | 274 - 275 |
| 6 | Pr | H | OMe | H | OMe | 240 - 241 |
| 7 | Bu | H | OMe | H | OMe | 228 - 230 |
| 8 | Bn | H | OMe | H | OMe | 236 - 237 |
| 9 | Pr | H | H | OMe | H | 199 - 201 |
| 10 | Bu | H | H | OMe | H | 145 - 146 |
| 11 | Bn | H | H | OMe | H | 249 - 251 |
| 12 | 4-picolyl | H | H | OMe | H | 288 - 289 |
| 13 | Pr | H | OMe | OMe | H | 219 - 221 |
| 14 | Pr | OMe | H | H | OMe | 271 - 272 |
| 15 | iso-Bu | H | OMe | H | OMe | 206 - 208 |
| 16 | Bn | H | H | H | H | 261 - 262 |
| 17 | 4-picolyl | H | H | H | H | 280 - 281 |
| 18 | 4-picolyl | H | OMe | H | OMe | 225 - 227 |
| 19 | Bn | OMe | H | OMe | H | 254 - 256 |
| 20 | EtOEt | H | OMe | H | OMe | 225 - 227 |
| 21 | 3-butenyl | H | OMe | H | OMe | 215 - 217 |
| 22 | 4-picolyl | H | COOMe | H | H | 250 - 251 |
| 23 | 4-Cl-Bn | H | H | H | H | 240 - 241 |
| 24 | 3-(2-Me-picolyl) | H | H | H | H | 286 - 288 |
| 25 | 2-picolyl | H | H | H | H | 236 - 237 |
| 26 | 3-picolyl | H | H | H | H | > 300 |
| 27 | 3-Cl-Bn | H | H | H | H | 230 - 231 |
| 28 | 4-MeO-Bn | H | H | H | H | 248 - 250 |
| 29 | 4-F-Bn | H | H | H | H | 224 - 226 |
| 30 | 4-Me-Bn | H | H | H | H | 224 - 225 |
| 31 | 3-NO₂-Bn | H | H | H | H | 247 - 248 |
| 32 | 2-Cl-Bn | H | H | H | H | 262 - 263 |
| 33 | 3-Me-Bn | H | H | H | H | 248 - 249 |
| 34 | 3,4-Cl₂-Bn | H | H | H | H | 245 - 246 |
| 35 | 3-MeO-Bn | H | H | H | H | 247 - 248 |
| 36 | 4-CF₃-Bn | H | H | H | H | 170 - 173 |
| 37 | 2-thienylmethyl | H | H | H | H | 279 - 282 |
| 38 | 2-furfuryl | H | H | H | H | 265 - 268 |
| 39 | 3-thienylmethyl | H | H | H | H | 276 - 280 |
| 40 | 3-(2-Cl-6-Me-picolyl) | H | H | H | H | 243 - 245 |
| 41 | 4-COOMe-Bn | H | H | H | H | 242 - 244 |
| 42 | 2-dioxoranylmethyl | H | H | H | H | 265 - 267 |
| 43 | 4-COOH-Bn | H | H | H | H | > 300 |
| 44 | Bn | H | Cl | H | H | 254 - 256 |
| 45 | 4-NO₂-Bn | H | H | H | H | 245 - 248 |
| 46 | 2-MeO-Bn | H | H | H | H | > 300 |
| 47 | 3,5-(MeO)₂-Bn | H | H | H | H | 224 - 225 |
| 48 | 2-(5-Cl-thienyl)-methyl | H | H | H | H | 251 - 254 |
| 49 | Bn | H | F | H | F | 253 - 255 |
| 50 | (1-naphthyl)-methyl | H | H | H | H | > 300 |
| 51 | 3,4-Me₂-Bn | H | H | H | H | 243 - 244 |
| 52 | Bn | H | OMe | H | H | 222 - 225 |
| 53 | 4-Br-Bn | H | H | H | H | 249 - 250 |
| 54 | 3-(2-Cl-picolyl) | H | H | H | H | 258 - 259 |
| 55 | Bn | H | OBn | H | H | 213 - 215 |
| 56 | 5-(3-Me-isoxazolyl)-methyl | H | H | H | H | 250 - 251 |
| 57 | 4,-(3,5- Me₂-isoxazolyl)-methyl | H | H | H | H | 253 - 254 |
| 58 | 5-Ph-pentyl | H | H | H | H | 135 - 137 |

| | | | | | | |
|---|---|---|---|---|---|---|
| * reference example | | | | | | |

### Example 2: Relaxing Action to Smooth Muscle of Airway of Guinea Pigs.

A guinea pig was killed by draining out the blood, airway was isolated, and four airway pieces having a width of about 1 cm cut along the cartilage were connected by silk yarn to prepare an airway smooth muscle sample. The sample was hung with a load cf about 0.5 g in a 5-mL Magnus vessel filled with a Tyrode solution and aerated with a mixed gas (95% O₂ and 5% CO₂).

After the sample was allowed to stand for 30 minutes to one hour, it was treated with histamine (final concentration: 10⁻⁵M) and the constriction was recorded on a recorder via an isotonic transducer. This was repeatedly treated with 10⁻⁵M of histamine and, after confirming that the constriction became constant, it was treated with 10⁻⁴ M of histamine.

After the maximum constriction reaction of the smooth muscle became constant, the test compound was added thereto starting from low concentration cumulatively to investigate the relaxing action. A dose vs. reaction curve was prepared from the isotonic reaction of various concentrations of the test compound and ED₅₀, a concentration showing 50% of the maximum reaction, was determined.

7-Amino-1,3-diethyl-1,2,3,4-tetrahydropyrido-[2,3-d]pyrimidine-2,4-dione (Control Compound A; a compound mentioned in the Japanese Laid-Open Patent Publication Hei-8/3046), 5-amino-1,3,-diethyl-1,2,3,4-tetrahydropyrido[2,3-d]pyrimidine-1,4-dione (Control Compound B; a compound mentioned in the Japanese Laid-Open Patent Publication Sho-63/45279) and theophylline were used as control agents.

An example of the result is shown in Table 2. The compounds of the present invention showed better efficacy than the known bronchial dilators when tested by a relaxing action to airway smooth muscle isolated from guinea pigs.

**Table 2**

| Compound No. | EC₅₀ (µM) |
|---|---|
| 5* | 1.7 |
| 6 | 0.40 |
| 7 | 0.59 |
| 8 | 0.32 |
| 15 | 0.21 |
| 16 | 0.023 |
| 17 | 0.019 |
| 18 | 0.061 |
| 28 | 0.045 |
| 29 | 0.076 |
| 37 | 0.021 |
| 38 | 0.040 |
| 39 | 0.027 |
| Control Compound A | 1.01 |
| Control Compound B | 0.83 |
| theophylline | 51 |

### Example 3: Influence on Continuous Constriction of Airway Smooth Muscle of Rats.

The back of the head of rat was struck to cause a cerebral concussion, carotid arteries on both sides were immediately cut and the pulmonary main artery was isolated. The isolated artery was fully aerated with a mixed gas (95% O₂ and 5% CO₂), placed in a Krebs-Henselite solution warmed at 37°C, excessive tissues were removed as much as possible and a spiral sample (having a width of about 2 mm and a length of 15 mm) was prepared according to a method of Furchgott, et al. The blood vessel sample was hung with a load of about 0.5 g in a 5-mL Magnus vessel filled with a Krebs-Henseleit solution and aerated with a mixed gas (95% O₂ and 5% CO₂).

After the sample was allowed to stand for 30 minutes to one hour, it was treated with noradrenaline (final concentration: 10⁻⁷ M) and the constricting reaction was amplified via an amplifier for blood pressure using an FD pickup and recorded on a recorder. Noradrenaline (10⁻⁷ M) was repeatedly applied and, after confirming that the constriction became constant, 10⁻⁷ M of noradrenaline was applied.

After the maximum shrinking reaction of the smooth muscle became constant and the test compound was added thereto starting from the low concentration cumulatively to investigate the relaxing action. A dose vs. reaction curve was prepared from the isotonic reaction of various concentrations of the test compound and an ED₅₀, a concentration showing 50% of the maximum reaction, was determined.

An example of the result is shown in Table 3. The compounds of the present invention showed little affection to blood vessel and high safety when tested by an influence on a continuous constriction of smooth muscle of blood vessel isolated from rats.

**Table 3**

| Compound No. | EC₅₀ (µM) |
|---|---|
| 5 | >100 |
| 6 | >100 |
| 15 | >100 |
| 16 | 50.1 |
| 18 | >100 |

### Example 4: Influence on General Symptoms of Mice.

Mice having no abnormality in their appearance before administration of the test drugs were selected, and five mice were used for each group. Oral administration of the test compound was carried out and, 30 minutes, one hour and two hours thereafter, observations were conducted according to the modified method of Irwin's method for observing the general symptoms of mice. Degree of the symptom was evaluated in terms of (+) and (-) and, when the symptom was apparently severe, it was mentioned as (++). Incidentally, death was observed until the next day of the administration.
- 1): Suppression of spontaneous motility: When mice were transferred from a home cage to a cage for symptom observation and, if motion of the mice was less than the non-administered group at that time, that was evaluated as (+).
- 2): Muscle relaxation: When forefeet of mice were hung on a wire stretched horizontally so that the mice were hung by means of the forefeet and, if their reaction at that time for climbing up the wire took longer time than the non-administered group, that was evaluated as (+).
- 3): Passiveness: When mice were hung by holding the neck of the mice between two fingers and, if the mice did not move so much at that time, that was marked as (+).
- 4): Blepharoptosis: If 1/4 or more was closed as compared with the non-administered group, that was evaluated as (+).
- 5): Salivation: If salivation was noted a little around the mouth, that was evaluated as (+).
- 6): Death: If dead case was noted, that was mentioned as such.

5-Amino-1,3-diethyl-1,2,3,4-tetrahydropyrido[2,3-d]-pyrimidine-2,4-dione (Control Compound B; a compound mentioned in the Japanese Laid-Open Patent Publication Sho-63/45279) was used as a control drug.

An example of the result is shown in Table 4. When the compounds of the present invention were tested in terms of an influence on the general symptoms of mice, they showed very high safety as compared with the known bronchial dilator having the similar structure.

**Table 4**

| Comp. No. | admin. dose (mg/kg) | Suppr. of spontaneous motility | Muscle relaxation | Passiveness | Blepharoptosis | Salivation | Death |
|---|---|---|---|---|---|---|---|
| 5 | 1000 | (+) × 2 | (+) × 3 | (-) | (+) × 3 | (-) | 0 |
| 6 | 300 | (+) × 1 | (-) | (-) | (+) × 1 | (-) | 0 |
| | 1000 | (+) × 2 | (+) × 2 | (-) | (+) × 2 | (-) | 0 |
| 7 | 300 | (+) × 2 | (+) × 2 | (-) | (+) × 1 | (-) | 0 |
| | 1000 | (+) × 1 | (+) × 3 | (-) | (-) | (-) | 0 |
| | | (++) × 1 | | | | | |
| Control Comp. B | 100 | (+) × 3 | (+) × 3 | (+) × 1 | (+) × 2 | (-) | 0 |
| | 300 | (+) × 3 | (+) × 4 | (+) × 1 | (+) × 2 | (-) | 0 |
| | | | (++) × 1 | (++) × 2 | (++) × 1 | | |

### Example 5: Concentration in Blood of Guinea Pigs.

Each test substance was orally administered at the dose of 30 mg/kg. It was suspended in 1% methyl cellulose and made into a preparation so as to make the administering dose 5 mL/kg.

A group consisted of four animals and blood was collected after 30 minutes, 1 hour, 2 hours, 4 hours, 6 hours and 24 hours from the administration (A feed was given after collection of the blood after 6 hours). Thus, each about 200 mL (corresponding to four capillary tubes) of blood were collected with intervals using a heparin-treated capillary tube and plasma was separated by a hematocrit centrifuge to prepare a plasma sample. A plasma sample of about 100 mL was preserved at -80°C until the measurement.

Methanol (200 mL) was added to 100 mL of the plasma followed by mixing, the mixture was centrifuged at 1500 × g for ten minutes at 40°C and the separated supernatant liquid was filtered through a membrane filter of 0.5 mm. The filtrate was used as a sample for an HPLC, analyzed under the analyzing condition that the column was 100 mm x 4.6 mm (inner diameter) of TSK-gel Super ODS, the flow rate was 1.0 mL/minute, the column temperature was 40°C, an injection amount was 6 mL, detection was done by UV of 225 nm and the mobile phase was water-acetonitrile (75:25 in terms of % by volume) and the maximum concentration in blood (Cₘₐₓ), time required for achieving the maximum concentration in blood (Tₘₐₓ), half life in blood (T_{½}) and area under a curve of concentration in blood vs. time (AUC 0-lim) were determined.

RS-25344 (*Cell Signal*, **7**(5), 527 (1995); *Mol. Pharmacol*., **48**(4), 616 (1995)) and CR-77059 (*J. Med. Chem.*, **34**, 624 (1991); *J. Pharmacol. Exp. Ther*, **272**, 3 (1995)) were used as control drugs.

An example of the result is shown in Table 5. When the behavior in blood of guinea pigs was tested, the compounds of the present invention showed a good transfer into blood and a long half life exhibiting a favorable behavior *in vivo* as compared with known xanthine-related compounds having similar structures.

**Table 5**

| Compound No. | Cₘₐₓ (µg/mL) | Tₘₐₓ (h) | T_{1/2} (h) | AUC 0-lim (µg h/mL) |
|---|---|---|---|---|
| 5 | 30.0 | 3.0 | 12.1 | 458.2 |
| 6 | 13.7 | 4.0 | 28.4 | 255.8 |
| 7 | 1.6 | 15.0 | 131.4 | 28.6 |
| 15 | 10.1 | 2.3 | 9.8 | 133.8 |
| RS-25344 | (less than identification limit) | | | |
| CP-77059 | (less than identification limit) | | | |

### Advantage of the Invention

As shown in Table 2, the 7-amino-1-phenylpyrido[2,3-d]pyrimidine-2,4-dione derivatives of the present invention exhibit better bronchial dilating action than the known bronchial dilators having the similar structures. Accordingly, the compounds of the present invention are useful as therapeutic agents for bronchial asthma.

It is also apparent from Tables 3 and 4 that the compounds of the present invention show far higher safety than the known bronchial dilators having the similar structures. It is further apparent from Table 5 that the compounds of the present invention show a good transfer into blood and a long half life in blood which are not available in the known xanthine-related compounds whereby a favorable behavior *in vivo* is achieved. Accordingly, the compounds of the present invention have far better characteristics as pharmaceuticals than the known compounds having the similar structures.

As mentioned above, the compounds of the present invention have an excellent bronchial dilating action, a high safety and little side effect whereby they are the compounds solving the problems in the prior art. Moreover, the compounds of the present invention exhibit a favorable behavior *in vivo* and their usefulness as pharmaceuticals is quite high.

## Claims

1. A 7-amino-1-phenylpyrido[2,3-d]pyrimidine-2,4-dione derivative of the formula (I) or a pharmaceutically acceptable salt or hydrate thereof 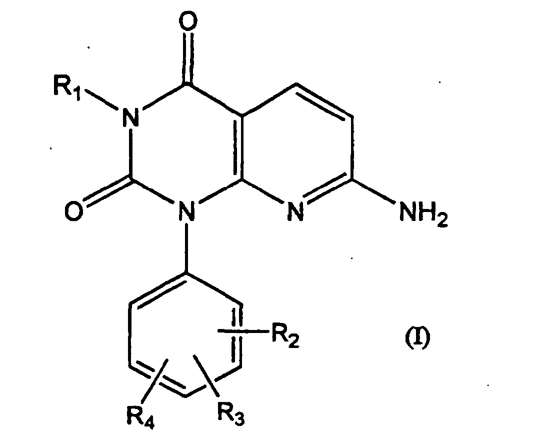 wherein R₁ is C₂₋₆-alkenyl, phenyl, C₃₋₆-alkyl or C₁₋₆-alkyl which is substituted with a substituent selected from
(a) oxo,
(b) C₁₋₆-alkoxy,
(c) phenyl which is optionally substituted with one or more C₁₋₆-alkyl, C₁₋₆-alkoxy, carboxyl, (C₁₋₆-alkoxy)carbonyl, mercapto, halogen, trifluoromethyl and/or nitro;
(d) naphthyl,
(e) furyl,
(f) isoxazolyl which is optionally substituted with one or more C₁₋₆-alkyl,
(g) pyridyl which is optionally substituted with one or more C₁₋₆-alkyl and/or halogen,
(h) thienyl which is optionally substituted with halogen, and
(i) 1,3-dioxolanyl;
and R₂, R₃ and R₄ each independently is hydrogen, halogen, C₁₋₆-alkoxy, benzyloxy, carboxyl or (C₁₋₆-alkoxy)carbonyl.

2. Compound according to claim 1, wherein R₂ is hydrogen.

3. Compound according to claim 1 or 2, wherein R₃ and/or R₄ is C₁₋₆-alkoxy.

4. Compound according to claim 3, wherein R₃ and/or R₄ is present in the meta-position of the aromatic ring.

5. Compound according to claim 3 or 4, wherein the C₁₋₆-alkoxy is methoxy.

6. Pharmaceutial composition comprising a 7-amino-1-phenylpyrido[2,3-d] pyrimidine-2,4-dione derivative according to any of the claims 1 to 5 as an effective component.

7. Use of a 7-amino-1-phenylpyrido[2,3-d] pyrimidine-2,4-dione derivative according to any of the claims 1 to 5 for the preparation of a medicament efective in the treatment of bronchial asthma or as a bronchial dilator

## Patentansprüche

1. 7-Amino-1-phenylpyrido[2,3-d]pyrimidin-2,4-dionderivat der Formel (I) oder ein pharmazeutisch akzeptables Salz oder Hydrat davon: 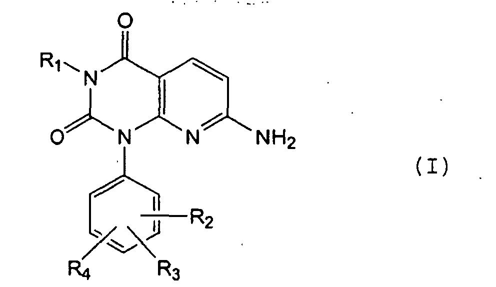 worin R₁ C₂₋₆-Alkenyl, Phenyl, C₃₋₆-Alkyl oder C₁₋₆-Alkyl ist, welches substituiert ist mit einem Substituenten ausgewählt aus
(a) Oxo,
(b) C₁₋₆-Alkoxy,
(c) Phenyl, welches gegebenenfalls substituiert ist mit einem oder mehreren C₁₋₆-Alkyl, C₁₋₆-Alkoxy, Carboxyl, (C₁₋₆-Alkoxy)carbonyl, Mercapto, Halogen, Trifluormethyl und/oder Nitro;
(d) Naphthyl,
(e) Furyl,
(f) Isoxazolyl, welches gegebenenfalls substituiert ist mit einem oder mehreren C₁₋₆-Alkyl,
(g) Pyridyl, welches gegebenenfalls substituiert ist mit einem oder mehreren C₁₋₆-Alkyl und/oder Halogen,
(h) Thienyl, welches gegebenenfalls substituiert ist mit Halogen, und
(i) 1,3-Dioxolanyl;
und R₂, R₃ und R₄ sind jeweils unabhängig voneinander Wasserstoff, Halogen, C₁₋₆-Alkoxy, Benzyloxy, Carboxyl oder (C₁₋₆-Alkoxy)carbonyl.

2. Verbindung gemäß Anspruch 1, worin R₂ Wasserstoff ist.

3. Verbindung gemäß Anspruch 1 oder 2, worin R₃ und/oder R₄ C₁₋₆-Alkoxy ist.

4. Verbindung gemäß Anspruch 3, worin R₃ und/oder R₄ in der meta-Position des aromatischen Rings vorliegt.

5. Verbindung gemäß Anspruch 3 oder 4, worin die C₁₋₆-Alkoxygruppe Methoxy ist.

6. Pharmazeutische Zusammensetzung umfassend ein 7-Amino-1-phenylpyrido[2,3-d]pyrimidin-2,4-dionderivat gemäß irgendeinem der Ansprüche 1 bis 5 als eine effektive Komponente.

7. Verwendung eines 7-Amino-1-phenylpyrido[2,3-d]pyrimidin-2,4-dionderivats gemäß irgendeinem der Ansprüche 1 bis 5 für die Herstellung eines Medikaments, welches wirksam ist in der Behandlung von Bronchialasthma oder als Bronchialdilatator.

## Revendications

1. Dérivé de 7-amino-1-phénylpyrido[2,3-d]pyrimidine-2,4-dione de formule (I) ou un de ses sels ou hydrates acceptables d'un point de vue pharmaceutique 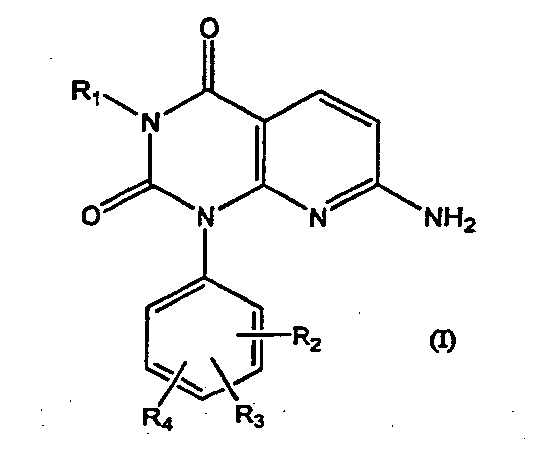 où R₁ est alcényle en C₂-C₆, phényle, alkyle en C₃-C₆ ou alkyle en C₁-C₆ substitué par un substituant choisi parmi
(a) oxo,
(b) alcoxy en C₁-C₆,
(c) phényle éventuellement substitué par un ou plusieurs groupes alkyle en C₁-C₆, alcoxy en C₁-C₆, carboxyle, (alcoxy en C₁-C₆)carbonyle, mercapto, halogéno, trifluorométhyle et/ou nitro;
(d) naphtyle,
(e) furyle,
(f) isoxazolyle éventuellement substitué par un ou plusieurs groupes alkyle en C₁-C₆,
(g) pyridyle éventuellement substitué par un ou plusieurs groupes alkyle en C₁-C₆ et/ou halogèno,
(h) thiényle éventuellement substitué par halogéno, et
(i) 1,3-dioxolanyle;
et R₂, R₃ et R₄ sont chacun hydrogène, halogéno, alcoxy en C₁-C₆, benzyloxy, carboxyle ou (alcoxy en C₁-C₆)carbonyle.

2. Composé selon la revendication 1, dans lequel R₂ est un hydrogène.

3. Composé selon la revendication 1 ou 2, dans lequel R₃ et/ou R₄ est alcoxy en C₁-C₆.

4. Composé selon la revendication 3, dans lequel R₃ et/ou R₄ est présent en position méta du cycle aromatique.

5. Composé selon la revendication 3 ou 4, dans lequel l'alcoxy en C₁-C₆ est méthoxy.

6. Composition pharmaceutique comprenant comme constituant efficace un dérivé de 7-amino-1-phénylpyrido[2,3-d]pyrimidine-2,4-dione selon l'une des revendications 1 à 5.

7. Utilisation d'un dérivé de 7-amino-1-phénylpyrido[2,3-d]pyrimidine-2,4-dione selon l'une des revendications 1 à 5 pour la préparation d'un médicament efficace dans le traitement de l'asthme bronchique ou comme bronchodilatateur.
